# EUROPEAN PATENT APPLICATION

(11) **EP 1 281 361 A1**
(43) Date of publication of application: **05.02.2003**
(21) Application number: 01500204.1
(22) Date of filing: 02.08.2001
(51) Int. Cl.: A61B 17/70

(54) **Device to prevent intervertebral disk degeneration**

(71) Applicant: Lafitt, S.A., 46988 Paterna (Valencia) (ES)
(72) Inventor: Forriol Pico, Joaquin, 46988 Paterna (Valencia) (ES)
(74) Representative: Isern-Cuyas, Maria Luisa

(57) **Abstract**

The "**Device to prevent intervertebral disk degeneration**" constituting the purpose of this Patent has a specific use after a surgical operation of back rachis fastening.

Its structure includes:
A connector (11) similar to a nut closed at one end, the screwed inside diameter (12) of which is adjusted to the upper screwed end of the bar (15) of the back fastener, whilst the closed head of the nut (11) is joined to the flexible cable (13).
A cable (13) made of a flexible material, that only works under traction, so that this is the load the set will transmit. One of its ends is joined to the connector (11) and the other to the bar (14).
A cylindrical longitudinal bar (14) coupled to the screws (1) by means of washers (4) and tightening screws (7), the length of which is adapted to the position of the screw (1).

## Description

### PURPOSE

The purpose to which the invention protected in this Patent refers is a "Device to prevent intervertebral disk degeneration" after an operation of back rachis fastening.

The device includes a set of mechanical means the structure of which has been designed with such an arrangement that permits, by means of fastening some of its component elements to the vertebras and to the bars of a back rachis fastener, preventing the degeneration of the upper intervertebral disks at a stabilized level by the fastening operation. The implantation of this device can be made during the back fastening operation or during a different operation.

Its function consists in preventing the stiff union of the damaged vertebral levels causing degeneration of the healthy upper disks which, if the device is not implanted, would be affected by the dynamic and kinematics alteration caused by the back fastener.

### HISTORY

There is no direct history of the "Device to prevent intervertebral disk degeneration", which is the purpose of this Patent, as until now there were no known devices the function of which was specifically degenerative prophylaxis after rachis back fastening operations.

The applicant company of this Patent is also the title holder of the Spanish Utility Model no. 98.00055, "Rachis stabilization device", requested on 13-01-98 and granted on 20.03.99, the implantation of which complements and improves the purpose of this Patent.

### DESCRIPTION OF THE INVENTION

The aim of the invention constituting the purpose of this Patent consists in the elimination of disadvantages pertaining to the known rachis stabilization devices, which result in the fact that the segments above those to be operated on suffer a degeneration due to the stiffness of the fastened vertebras.

For this reason the structure of said device has been designed to include the following elements as a minimum:
Two retention elements that are applied to the vertebra above the vertebras operated with the back fastener.
Two connection elements between the device and the closest ends of the bar of the back fastener.
Two longitudinal elements that transmit traction loads only. One of the ends is joined to the connection element with the bar and the other is joined to the retention element applied on the vertebra above the vertebras operated with the back fastener.

The described structure corresponds to the efficient minimum and the number of operated adjacent levels may be increased, with the corresponding increase in retention, connection and longitudinal elements that only transmit traction loads. The described structure for a sole upper level can be reproduced as many times as necessary, depending on the number and situation of the levels adjacent to the back fastener that are going to be operated.

The way in which the "Device to prevent intervertebral disk degeneration", constituting the purpose of this Patent, is able to overcome the disadvantages of the known rachis stabilization devices is as follows:

In a rachis back fastener, the operated levels form a stiff set, whilst the upper level maintains all its mobility. The stiffness of the lower segments (that have lost part of the capacity to absorb loads) results in that this upper level is subjected to loads greater than normal loads. Therefore, the upper disk suffers degenerative changes in its internal structure, caused by the new biomechanical conditions imposed by the back fastener.

To prevent this degeneration, it has been planned to apply the back fastener at a higher level also. This is not a satisfactory solution due to two reasons:
- The fastening and subsequent fusion implies a decrease in mobility, unavoidable in the damaged disks but of a doubtful usefulness in health disks.
- The problem would be repeated in the disk above the last one operated.

The invention that constitutes the purpose of this Patent introduces an intermediate solution between the degeneration and functional annulment of a healthy disk. To compensate the tensional changes caused by a back fastener, this invention proposes a flexible fastening between the operated levels and the upper level, so that it modifies (but does not annul) the load transmission by means of a flexible cable that does not prevent movement but reduces it to normal conditions. In short, a shock absorber of the stresses caused by the most important movements of the column (front-back flexion, side flexion and torsion), in such a way that this shock absorbing compensates the increase caused by the stiffness of the back fastener. This compensation will return the tensional normality to the upper disk, preventing its degeneration and maintaining its mobility.

The function of each element of the invention as regards the above described overall purpose is as follows: Longitudinal element that only transmits traction loads: To transmit a traction force between the upper level and the operated levels, compensating the stiffness of the latter.

Connection element: To transmit the load from the bar of the back fastener used to the longitudinal element made of a flexible material.

### BRIEF DESCRIPTION OF THE DRAWINGS

To complement the description of the invention and facilitate the interpretation of the formal, structural and functional characteristics of its purpose, attached are drawings in which different aspects of a preferred performance of the "Device to prevent intervertebral disk degeneration", constituting the purpose of this Patent, are schematically represented.

In said drawings:

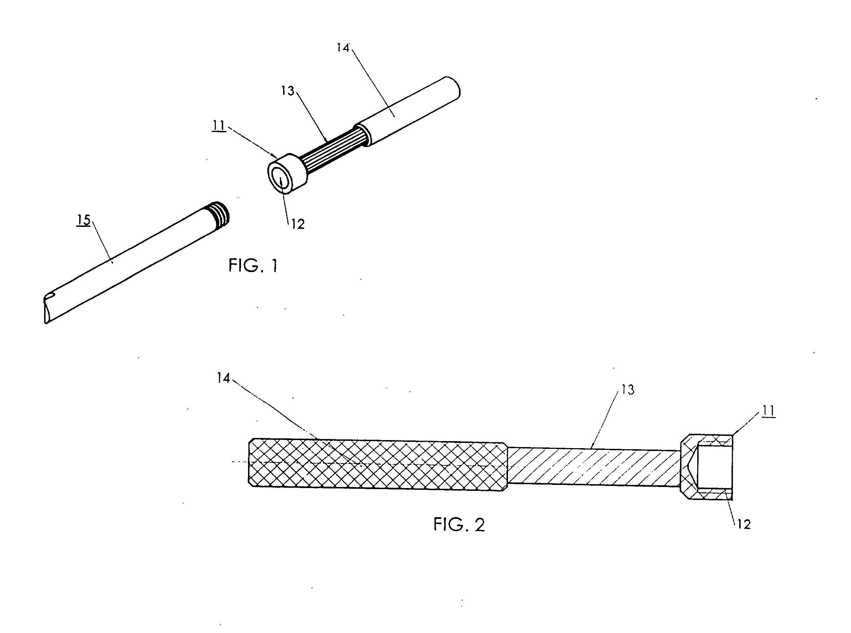

Figure 1 represents a perspective view of the structure of the device, showing the informative situation of its component elements, as well as the bar of the back fastener with the screwed end to which the device is applied.
Figure 2 represents a longitudinal section of the connector-flexible cable-bar set.

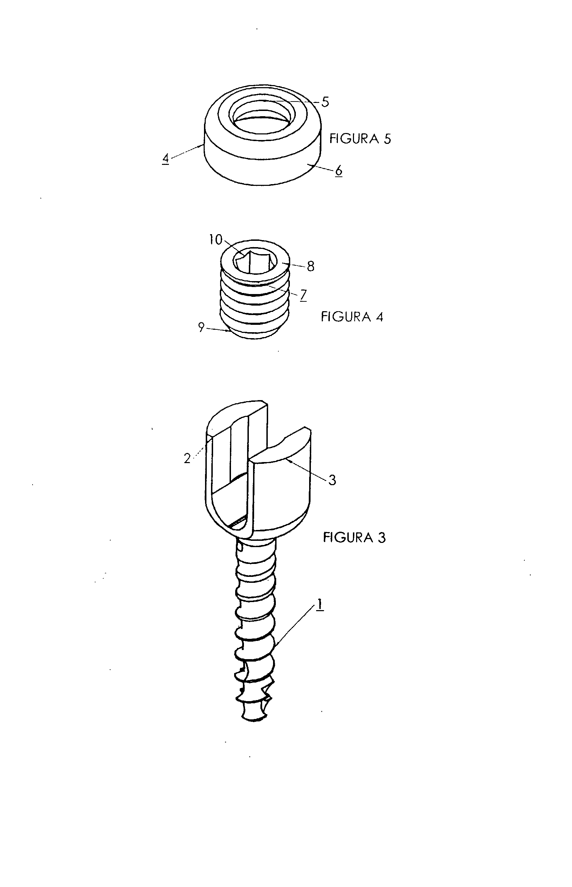

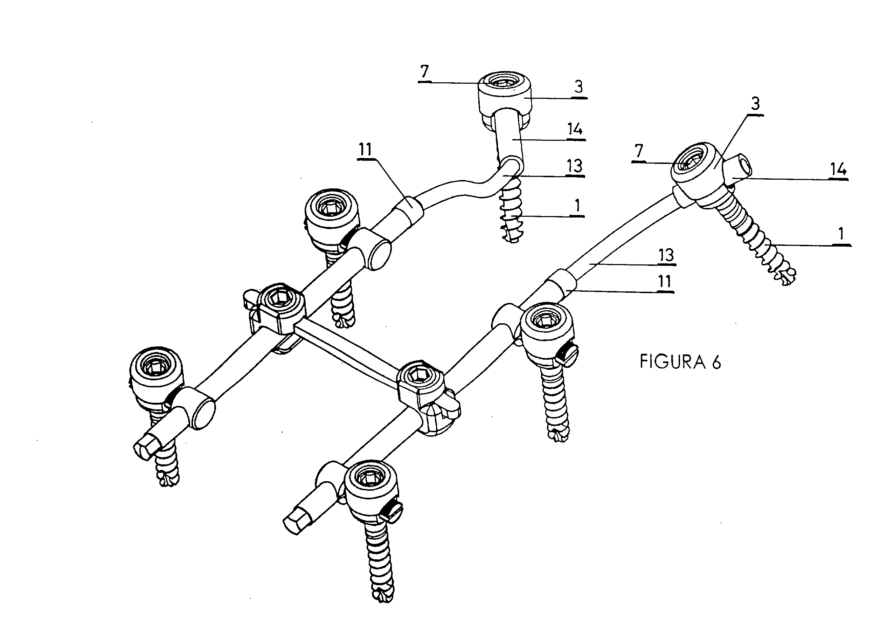

Figure 3 is a perspective view of a fastening screw to the vertebras; figure 4 of the tightening screw and figure 5 of the double diameter washer.
Figure 6 shows a perspective view of the set of a back rachis fastener where devices constituting the purpose of this Patent have been coupled to the upper ends of its longitudinal bars.

### DESCRIPTION OF A PREFERRED PERFORMANCE

To dearly show the nature and scope of the advantageous application of the "Device to prevent intervertebral disk degeneration", purpose of the claimed invention, the following is a description of its structure and the characteristics of its component elements, making reference to the drawings which, on representing a preferred performance of said purpose for information, must be considered in the widest sense and not limiting of the application and content of the claimed invention.

As this device completes the surgical action of a back fastener, this preferred performance may be used with any back fastener.

The minimum efficient structure of the "Device to prevent intervertebral disk degeneration" constituting the purpose of this Patent consists of: Two screws (1) that will be inserted into the vertebra above the ones operated with the back fastener, with a diameter and longitudinal less than the transpedicular screws of the back fastener, as they will only support traction loads and thus making it unnecessary to use such an aggressive fastening as that of said transpedicular screws. The head (3) has a wide U-shaped groove with the bottom cogged and the side curved walls (2) screwed internally.
- Two tightening washers (4) with two distinct inside diameters, the largest and lowest (6) adjusted to the outside diameter of the head (3) of the screws (1) to which it is coupled; and the smallest and highest (5) constituting a step in which the head (3) of the screws (1) makes a butt, thus limiting its penetration, and with an inside screw thread.
- Two tightening screws (7) with the diameter lateral cylindrical surface adjusted to the smallest and highest (5) of the double diameter washers **(4) and screwed on them, finished on the lower part with the shape of an** inverted truncated cone (9) and provided with an axial hexagonal cavity (10) accessible on the upper part to facilitate working the wrench (tightening or loosening).

All the above described elements (screws, washers and tightening screws) and their characteristics are protected in the Spanish M.U. no. 9800055, with the same title as this Patent. For this reason they are described herein but are not claimed.

Two connector-flexible cable-bar sets that transmit traction loads of the transversal fastener to the screw of the adjacent upper level and consist of the following elements each one:
A connector (1) similar to a nut closed at one end, the screwed inside diameter (12) of which is adjusted to the screwed upper end of the bar (15) of the back fastener, whilst the closed head of the nut (11) is joined to the flexible cable (13).
A cable (13) made of a flexible material that only works under traction, so that this is the load the set will transmit. One of its ends is joined to the connector (11) and the other to the bar (14).
A cylindrical longitudinal bar (14) coupled to the screws (1) by means of washers (4) and tightening screws (7), the length of which is adapted to the position of the screw (1).

The described structure corresponds to the efficient minimum and the number of adjacent or non-adjacent operated levels may be increased with the corresponding increase in screws (1), washers (4) and tightening screws (7); and with a connector-flexible cable-bar set that reproduces the described structure for a unique upper level as many times as necessary.

## Claims

1. Device to prevent intervertebral disk degeneration after a back rachis fastening operation, **characterized by** the fact that its efficient minimum structure consists of: Two connector-flexible cable-bar sets that transmit tractions loads from the transversal fastener to the adjacent upper level screw which are composed of the following elements each one: *A connector (11) similar to a nut closed at one end, the screwed inside diameter (12) of which is adjusted to the inside diameter of the screwed upper end of the bar (15) of the back fastener, whilst the closed head of the nut (11) is joined to the flexible cable (13); *a cable (13) made of a flexible material that only works under traction, so that this is the load the set will transmit. One of its ends is joined to the connector (11) and the other to the bar (14); a cylindrical longitudinal bar (14) coupled to the screws (1) by means of washers (4) and tightening screws (7), the length of which is adapted to the position of the screw (1).

2. Device to prevent intervertebral disk degeneration, according to claim 1, **characterized by** the fact that the number of adjacent or non-adjacent operated levels can be increased, with the corresponding increase in screws (1), washers (4) and tightening screws (7); and with a connector-flexible cable-bar set that reproduces the described structure for a unique upper level as many times as necessary.
